# EUROPÄISCHE PATENTSCHRIFT

(11) **EP 1 182 973 B1**
(45) Veröffentlichungstag und Bekanntmachung des Hinweises auf die Patenterteilung: **29.12.2004**
(21) Anmeldenummer: 00943781.5
(22) Anmeldetag: 07.06.2000
(51) Int. Cl.: A61B 17/12

(54) **FISTELBLOCKER**
FISTULA BLOCKER
OBTURATEUR DE FISTULE

(30) Priorität: 07.06.1999 DE 29909888 U
(43) Veröffentlichungstag der Anmeldung: 06.03.2002
(73) Patentinhaber: Novomed GmbH, 67655 Kaiserslautern (DE)
(72) Erfinder: BURGARD, Gunther, D-66424 Homburg (DE)
(74) Vertreter: Grünecker, Kinkeldey, Stockmair & Schwanhäusser Anwaltssozietät
(86) Internationale Anmeldenummer: PCT/EP2000/005273
(87) Internationale Veröffentlichungsnummer: WO 2000/074576

(56) Entgegenhaltungen:
- EP-A- 0 894 474
- WO-A-89/11301
- WO-A-94/28800
- WO-A-96/04954
- WO-A-98/06344
- FR-A- 2 625 897
- US-A- 5 374 261

## Beschreibung

Die Erfindung bezieht sich auf einen Fistelblocker zum Sanieren eines Fistelganges, insbesondere zum Behandeln von Anal-, Blasen-, Darm- und Urogenitalfistein.

Fisteln sind röhrenförmige, mit Gewebe ausgekleidete Verbindungen zwischen Körperhöhlen bzw. Hohlorganen untereinander oder der Körperoberfläche. Sie entwickeln sich häufig in Folge von Infektionen oder gehen mit Abszessbildung einher. Zum Beispiel entstehen Analfisteln vorwiegend aus einer Infektion der sogenannten Proktodäaldrüse. Analfisteln bilden in der Regel eine Verbindung zwischen dem Dickdarmende (Rektum) und der äußeren Haut der Pobacke. Dabei durchsetzen sie häufig die Schließmuskulatur.

In herkömmlicher Weise wird die Mehrzahl der Analfisteln durch die sogenannte "lay-open"-Technik behandelt. Dabei wird die Fistel vorsichtig sondiert und das darüber liegende Gewebe, zumeist mit der Schließmuskulatur (Sphinktermuskulatur) durchtrennt. Die Durchtrennung wird soweit vorangetrieben bis der Grund der Trennstelle durch den längsgespaltenen Gang der Fistel gebildet wird. Diese Trennstelle yerheilt von unten nach oben, so dass sich die Fistel schließt. Wegen der Durchtrennung der Sphinktermuskulatur besteht jedoch die Gefahr einer nachfolgenden Inkontinenz des Patienten.

In der SU 1 718 837 A1 ist ein Verfahren zum Behandeln von Dickdarmfistein beschrieben, welche beispielsweise den Dickdarm mit der Bauchdecke verbinden. Dabei wird ein Draht von außen bis zur inneren Öffnung der Fistel eingeführt. Ein Obturator wird mit Hilfe eines Endoskops durch den Dickdarm zur inneren Öffnung geführt. Der Obturator wird an dem Drahtende befestigt und zum Verschließen in die innere Öffnung des Fistelkanals gezogen. Anschließend wird der Draht entfernt und die äußere Öffnung mit Hilfe eines separaten Obturationselement verschlossen. Auf diese Weise wird der Fistelgang eliminiert.

Die SU 1 204 193 A offenbart einen Obturator zum Verschließen von bronchiopleuralen Fisteln nach Pneumektomiebehandlungen. Der Obturator hat eine Kegelstumpfform mit einer Öffnung an dem rückseitigen Ende. In die rückseitige Öffnung wird eine Knopfsonde eingeführt, mit Hilfe welcher der Obturator in eine Fistelöffnung eingeführt wird, nachdem seine Oberfläche und die Fistelwände mit medizinischem Klebstoff versehen wurden. Mit Hilfe des Klebstoffes wird der Obturator an der Fistelwand fixiert und die Knopfsonde wird anschließend wieder entfernt.

Die DE 26 37 119 A1 schlägt als Verschlusskörper zum Verschließen von Blutgefäßen oder Fisteln nach operativen Eingriffen einen aufblasbaren Ballon vor, welcher in das entsprechende Gefäß eingebracht werden kann, dort aufgeblasen wird und verbleibt. Nach dem Platzieren und Aufblasen des Ballons wird eine Schlauchleitung zum Aufblasen des Ballons von dem Ballon getrennt und aus dem Körper herausgezogen.

Die Druckschrift WO89/11301 beschreibt einen schwammartigen Verschlusskörper zum Verschließen von Punktionen oder Inzisionen nach Gefäßoperationen, insbesondere in Blutgefäßen. Der Verschlusskörper wird mit Hilfe einer Hülse, welche z. B. bereits für einen Katheter verwendet wurde, in das Blutgefäß eingebracht und von innen her in den Durchgang gezogen. Zum Einziehen steht ein Faden zur Verfügung, welcher nach außen gezogen und befestigt wird und dessen Material nach gewisser Zeit im Körper zerfällt.

Der Erfindung liegt die Aufgabe zugrunde, eine Behandlungsvorrichtung zum Sanieren von Fisteln zu schaffen, mit der Fisteln möglichst schonend behandelt werden können, wobei die Funktionen der anliegenden anatomischen Strukturen möglichst erhalten bleiben sollen.

Diese Aufgabe wird erfindungsgemäß gelöst mit einem Fistelblocker zum Sanieren eines Fistelganges, mit einem stöpselartigen, wenigstens ansatzweise in einen Fistelgang einführbaren Verschlusskörper, welcher eine sich mindestens teilweise umfangsseitig, quer zur Einführrichtung erstreckende und in Kontakt mit der Wand eines Fistelgangs bringbare Anlagefläche aufweist, wobei der Verschlusskörper mit einem flexiblen, in den Fistelgang einführbaren Applikationsstrang versehen ist, welcher als Drainageleitung ausgebildet ist

Dieser Fistelblocker ermöglicht eine wesentliche Verbesserung der Operationstechnik. Nachdem die Fistel sondiert wurde, kann der Fistelblocker in eine Öffnung des Fistelganges eingeführt werden und in gewünschter Tiefe platziert werden. Mit Hilfe des Verschlusskörpers wird der Fistelgang an einer Seite weitgehendst verschlossen, so dass Erreger von dieser Seite her von dem Verschlusskörper blockiert werden. Die Anlagefläche des Verschlusskörpers befindet sich in eingeführtem Zustand in Kontakt mit der Wand des Fistelganges, wodurch eine gewisse Dichtwirkung entsteht.

Der Applikationsstrang kann in den Fistelgang eingeführt werden und wahlweise den Verschlusskörper nach sich in den Fistelgang ziehen. Durch die Ausbildung als Drainageleitung kann der Applikationsstrang zumindest zeitweise in der Fistel verbleiben. Sekrete oder Eiterflüssigkeit können über die Drainageleitung aus dem Fistelgang abgeleitet werden, so dass Entzündungsherde nach außen geführt werden. Als Drainageleitung ist z. B. ein Drainagefaden denkbar, an dem keimbildende Sekrete durch eine dochtartige Wirkung aus dem Fistelgang nach außen geführt werden.

Der erfindungsgemäße Fistelblocker ermöglicht eine äußerst schonende Behandlung der Fistel, insbesondere für das umliegende Gewebe. Während im Stand der Technik das umliegende Gewebe bis zum Grund des Fistelganges durchtrennt wurde, ist mit dem Fistelblocker eine schonende Behandlungstechnik möglich, welche für den Patienten wesentlich verträglicher ist und die umliegenden anatomischen Strukturen kaum beeinflusst.

Vorzugsweise kann der Verschlusskörper einen in Einführrichtung kranial angeordneten Führungsabschnitt aufweisen. Der Führungsabschnitt erleichtert das Einführen des Verschlusskörpers, wobei er den Verschlusskörper dem anatomischen Verlauf des Fistelganges entsprechend ausrichtet.

Besonders vorteilhaft kann der Verschlusskörper einen in Einführrichtung kaudal angeordneten, die Anlagefläche aufweisenden Schließabschnitt aufweisen. Mit Hilfe des Schließabschnitts erfolgt das Blocken des Fistelganges. Der Schließabschnitt liegt vorzugsweise mit der Anlagefläche an der Wandung des Fistelganges.

Günstigerweise kann der Verschlusskörper nach kranial konisch verjüngt ausgebildet sein. Die konische Form ermöglicht ein leichteres Einführen des Verschlusskörpers mit dem verjüngten führenden Ende. Beim Voranschieben des Verschlusskorpers wird der Fistelgang leicht gespreizt, womit der Fistelgang entfaltet wird und sich der Verschlusskörper mit Hilfe der Anlagefläche relativ dicht an die Wand der Fistel anlegt.

Als Variante der Erfindung kann der Verschlusskörper etwa kegelförmig ausgebildet sein. Die Kegelform hat einen sich kontinuierlich erweiternden Außendurchmessers des Verschlusskorpers, so dass er beim Einführen in den Fistelgang in gewünschter Tiefe bis zu dem den Fistelgang entsprechenden Durchmesser in die Fistelöffnung eingeschoben werden kann.

Möglicherweise kann der Verschlusskörper eine konkave Außenform aufweisen. Dies ist insbesonders günstig mit einer sich nach kaudal hin konkav erweiternden Querschnittsform des Verschlusskörpers, in deren Bereich vorzugsweise die Anlagefläche angeordnet ist, so dass sich diese besonders gut in die Fistelöffnung einführen läßt bzw. sich von außen an die Umgebung der Fistelöffnung anlegt, bis der Fisteldurchgang geblockt ist.

Als Variante der Erfindung kann der Verschlusskörper etwa eiförmig ausgebildet sein.

Denkbar kann der Verschlusskörper rotationssymmetrisch zu seiner Längsachse ausgebildet sein.

Gemäß einer bevorzugten Ausführungsform kann die Länge des Verschlusskörpers in Einführrichtung etwa bis 2 cm, vorzugsweise 0,5 bis 1 cm, entsprechen. Mit dieser Länge lässt sich ein Fistelgang wirksam blocken. Die Anlagefläche kann in Einführrichtung genügend lang ausgebildet sein, um die Fistel wirksam zu blocken. Ein Verschlusskörper dieser Länge ist von einem Patienten über längere Zeit problemlos tragbar.

Vorzugsweise kann der Verschlusskörper aus resobierbarem Material bestehen. Damit kann das Material des Verschlusskörpers über einen gewissen Zeitraum hin abgebaut werden. Während der Verschlusskörper anfänglich noch die Fistel blockt, wird er über den Lauf der Zeit langsam vom Körper umgebaut und je nach Materialart am Ende sogar ganz aufgelöst werden.

Besonders vorteilhaft kann der Verschlusskörper aus Polydioxanon, Polyglykolsäure und/oder Trimethylcarbonat bestehen. Diese Materialien sind langzeitresobierbar und können vom Körper langsam selbst abgebaut und aufgenommen werden.

Besonders günstig kann der Verschlusskörper aus Metall, vorzugsweise Titan bestehen.

Besonders vorteilhaft kann der Verschlusskörper im Inneren hohl ausgebildet sein. Damit wird nur relativ wenig Fremdmaterial in den Fistelgang eingesetzt. Wenn das hohle Gebilde im Inneren des Verschlusskörpers von außen zugänglich ist, kann der Verschlusskörper von körpereigenem Gewebe durchsetzt werden, was bei resorbierbarem Material die Granulation fördert.

Es wird vorgeschlagen, dass der Verschlusskörper eine semipermeable, in Richtung von kranial nach kaudal durchlässige Oberflächenstruktur, vorzugsweise als Membran, aufweist. Damit können Substanzen, z.B. Eiter und Flüssigkeit, durch den Verschlusskörper hindurch von kranial nach kaudal aus dem Fistelgang abgeleitet werden, während das Eindringen von Verunreinigungen von außen unterbunden wird.

Möglicherweise kann der Verschlusskörper eine schwammartige Struktur aufweisen. Dies erleichtert die Resorbtion und Granulation des Verschlusskörpers. Bei einer schwammartigen Oberflächenstruktur des Verschlusskörpers können körpereigene Stoffe besonders leicht in den Verschlusskörper eindringen und diesen umbauen bzw. über längere Zeit zersetzen.

Als Variante der Erfindung kann der Verschlusskörper im Inneren mit Kanälen durchsetzt sein. Dies begünstigt die Resorbtion des Verschlusskörpers.

Günstigerweise kann der Verschlusskörper mehrere über seine Oberfläche verteilte Vertiefungen aufweisen. Die Vertiefungen erhöhen die Griffigkeit des Verschlusskörpers, so dass dieser besser an der Wand des Fistelganges arretiert ist. Ferner begünstigen die Vertiefungen, z. B. in Form von Grübchen, die Resorption.

Gemäß einer besonderen Ausführungsform kann der Fistelblocker mit einer den Verschlusskörper in einem Fistelgang arretierenden Verankerungseinrichtung versehen sein. Damit kann der Fistelblocker im Körper auch bei Bewegungen des Patienten sicher befestigt werden.

Denkbar kann die Verankerungseinrichtung ein oder mehrere die Bewegung entgegen der Einführrichtung blockierende Wiederhakenelement/e aufweisen. Über die Widerhakenelemente wird die Bewegung entgegen der Einführrichtung gehemmt, so dass die Verankerungseinrichtung nicht ungewollt aus der Fistel herausrutscht.

In besonderer Weise können die Widerhakenelemente begrenzt bewegbar, sich selbst seitlich von dem Fistelblocker abspreizend gelagert sein. Damit stellen sich die Widerhakenelemente selbst seitlich aus und hemmen eine der Einführrichtung entgegengesetzten selbständigen Bewegung des Fistelblockers.

Um einen erfindungsgemäßen Fistelblocker der jeweiligen Größe und Anatomie des zu behandelnden Fistelganges auszuwählen und einen guten Sitz des Fistelblockers zu erreichen, können erfindungsgemäß separate Fistelblockerschablonen verwendet werden. Diese Fistelblockerschablonen zum Einsetzen in einen Fistelgang haben einen stöpselartigen, wenigstens ansatzweise in einen Fistelgang einführbaren Verschlusskörper, welcher eine sich mindestens teilweise umfangsseitig, quer zur Einführrichtung erstreckende und in Kontakt mit der Wand eines Fistelganges bringbare Anlagefläche aufweist. Diese Fistelschablonen können in verschiedenen Größen und Formen zur Verfügung stehen, so dass nacheinander mehrere verschiedene Verschlusskörper probeweise am Patienten angesetzt werden können und dementsprechend ein Fistelblocker passender Größe und Form ausgewählt und letztendlich eingesetzt wird.

Die Fistelblockerschablonen können vorzugsweise einen Verschlusskörper aufweisen, welcher die zuvor genannten spezifischen Merkmale des Verschlusskörpers des Fistelblockers aufweisen.

Ausführungsbeispiele der Erfindung sind in der Zeichnung dargestellt und werden nachstehend erläutert. Es zeigen:
- Fig. 1: eine Seitenansicht eines erfindungsgemäßen Fistelblockers gemäß einer ersten Ausführungsform,
- Fig. 2: eine vergrößerte Seitenansicht eines erfindungsgemäßen Fistelblockers gemäß einer zweiten Ausführungsform,
- Fig. 3: eine vergrößerte Seitenansicht eines erfindungsgemäßen Fistelblockers gemäß einer dritten Ausführungsform,
- Fig. 4: einen Längsschnitt durch ein menschliches Rektum mit angrenzenden anatomischen Strukturen und einem eingesetzten erfindungsgemäßen Fistelblocker und
- Fig. 5: eine vergrößerte Darstellung des rechten Teils von Figur 4 mit eingesetztem erfindungsgemäßen Fistelblocker.

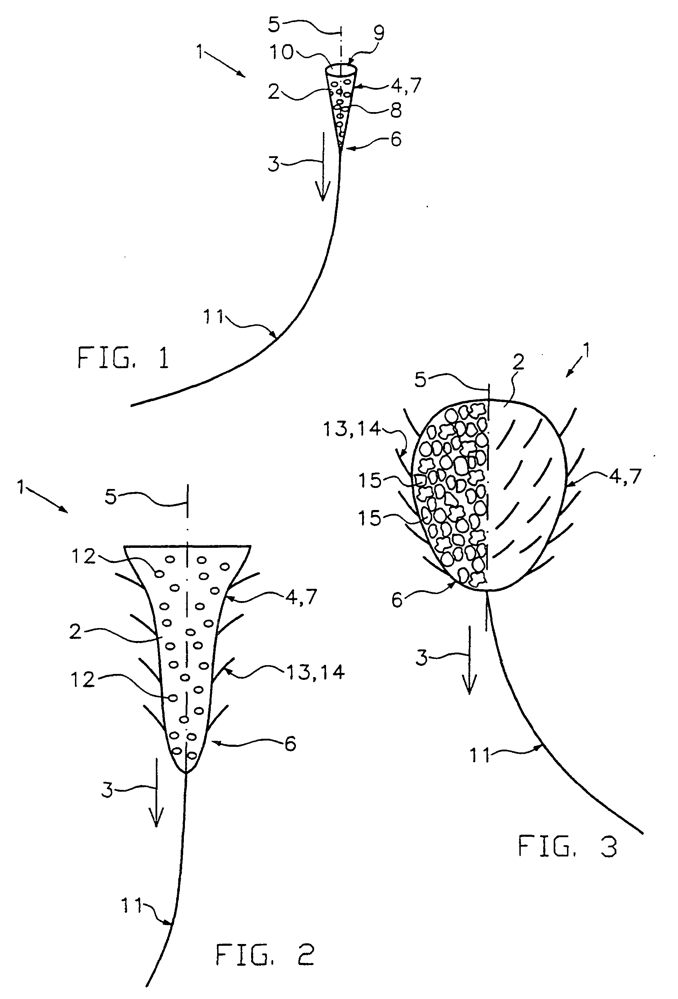

ln Figur 1 ist eine erste Ausführungsform eines erfindungsgemäßen Fistelblockers 1 zum Sanieren eines Fistelgangs dargestellt. Der Fistelblocker hat einen stöpselartigen Verschlusskörper 2, welcher in einer Einführrichtung 3 in einen Fistelgang einführbar ist. Der Verschlusskörper 2 hat eine Kegelform mit einem kaudalen dickeren Ende und einem kranial dünneren Ende. Das kaudale Ende bezeichnet in diesem Sinn das der Einführrichtung 3 entgegengesetzte Ende, während das kraniale Ende das in Einführrichtung 3 weisende Ende des Verschlusskörpers 2 ist.

Der Verschlusskörper 2 hat eine sich quer zur Einführrichtung 3 erstreckende Anlagefläche 4, welche bei der Kegelform durch die Kegelmantelfläche gebildet ist. Diese Fläche ist wenigstens bereichsweise in Kontakt mit der Wand eines Fistelgangs bringbar. Die Kegelform ist rotationssymmetrisch zu der Längsachse 5 des Verschlusskörpers 2.

Das kraniale Ende des Verschlusskörpers bildet einen Führungsabschnitt 6 und das kaudale Ende des Verschlusskörpers 2 bildet einen Schließabschnitt 7, welcher die Anlagefläche 4 enthält und bei eingeführtem Fistelblocker 1 in Kontakt mit der Wand des Fistelganges ist.

Die äußere Oberfläche des Verschlusskörpers weist mehrere grübchenartige Vertiefungen 8 auf. Der Verschlusskörper 2 hat innenseitig einen etwa kegelförmigen Hohlraum 9, welcher endseitig mit einer Wand 10 verschlossen ist.

An seinem konisch zulaufenden kranialen Ende ist der Verschlusskörper 2 mit einem Applikationsstrang 11 verbunden, welcher flexibel und in einen Fistelgang einführbar ist. Der Applikationsstrang ist als Drainagefaden ausgebildet, so dass er zum Ableiten von Flüssigkeiten aus der Fistel dienen kann. Der Applikationsstrang kann eine Länge von etwa 20 cm haben.

Der Verschlusskörper 2 hat außenseitig eine semipermeable Oberflächenstruktur, welche in Richtung von kranial nach kaudal durchlässig ist und in umgekehrter Richtung undurchlässig ist. Seine Oberfläche kann eine entsprechende Membran aufweisen bzw. der gesamte Verschlusskörper 2 kann membranartig ausgebildet sein.

In Figur 2 ist eine zweite erfindungsgemäße Ausführung eines Fistelblockers 1 dargestellt. Gleiche Bezugszeichen bezeichnen gleiche Elemente, so dass diesbezüglich auf die vorstehenden Ausführungen verwiesen wird, sofern die folgende Beschreibung keine davon abweichende Erläuterung gibt.

Der Verschlusskörper des Fistelblockers 1 gemäß der zweiten Ausführungsform hat einen Schließabschnitt 7 mit sich kaudal nach außen erweiternder konkaver Außenform. Mit der äußeren Anlagefläche 4 kann der Fistelblocker 1 in Kontakt mit dem Fistelgang gelangen. Der kraniale Führungsabschnitt 6 des Verschlusskörpers 2 hat eine nach vorne einwärts etwa konvex gewölbte Form, welche zu dem Applikationsstrang 11 hinführt. Somit hat der Verschlusskörper 2 im Längsschnitt etwa eine Glockenform.

Der Verschlusskörper 2 ist im Inneren mit Kanälen durchsetzt, welche über Kanalöffnungen 12 an die äußere Oberfläche des Verschlusskörpers 2 grenzen.

Der Verschlusskörper 2 ist mit einer Verankerungseinrichtung 13 versehen, welche mehrere längs des Umfangs und der Länge des Verschlusskörpers 2 verteilte Widerhakenelemente 14 aufweist. Die Widerhakenelemente 14 zweigen seitlich abgespreizt von dem Verschlusskörper 2 ab und sind etwa in Richtung entgegen der Einführrichtung 3 gerichtet. Sie sind begrenzt flexibel.

In Figur 3 ist eine dritte Ausführungsform eines erfindungsgemäßen Fistelblockers 1 dargestellt. Gleiche Bezugszeichen bezeichnen gleiche Elemente wie in den vorgenannten Ausführungsformen, so dass diesbezüglich auf die vorgenannte Beschreibung verwiesen wird, sofern nicht im folgenden davon abgewichen wird.

Der Verschlusskörper 2 hat etwa eine Ei-Form, deren schlankeres Ende in Einführrichtung 3 gerichtet ist. Der Verschlusskörper 2 hat eine schwammartige hohle Struktur, die im Querschnitt in der linken Hälfte des Verschlusskörpers 2 geschnitten dargestellt ist. Die schwammartige Struktur ist in Form von mehreren Poren 15 dargestellt, welche sich bis zur äußeren Oberfläche des Verschlusskörpers 2 erstrecken. Auch die Oberfläche des Verschlusskörpers 2 hat eine entsprechende spongiosaartige Struktur. Dementsprechend hat der Verschlusskörper im Inneren eine poröse hohle Struktur.

In der rechten Hälfte ist der Verschlusskörper 2 von außen dargestellt, wobei aus Übersichtlichkeitsgründen die schwammartige Struktur weggelassen wurde. Außenseitig hat der Verschlusskörper 2 ebenfalls eine entsprechende Verankerungseinrichtung 13 mit Widerhakenelementen 14.

Die Fistelblocker 1 der ersten bis dritten Ausführungsform haben Verschlusskörper 2 mit einer in Einführrichtung gemessenen Länge von etwa 2 cm, vorzugsweise 0,5 bis 1 cm. Sie können aus resorbierbarem Material bestehen, z.B. Polydioxanon, Polyglykolsäure und/oder Trimethylcarbonat.

Ebenso kann der Verschlusskörper aus nicht resorbierbarem Material, wie z.B. Metall, vorzugsweise Titan, bestehen.

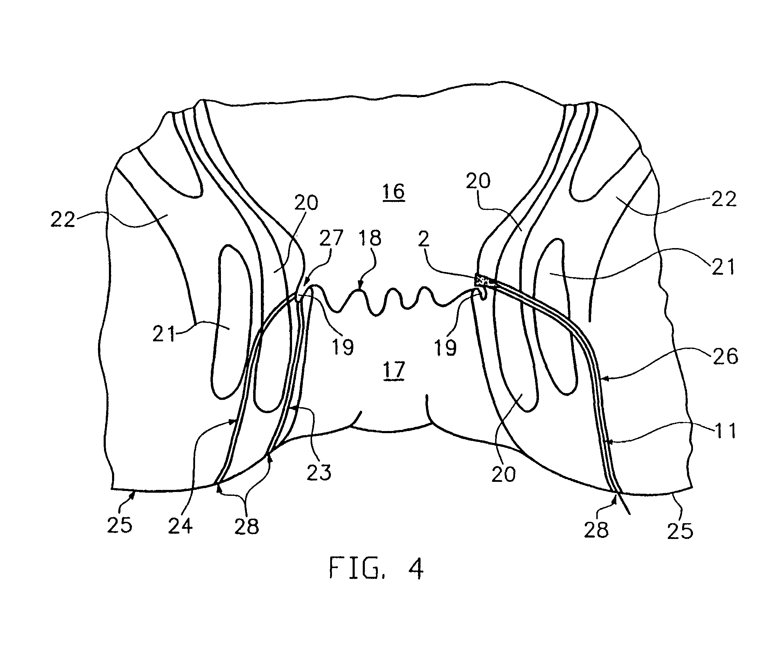

In Figur 4 ist ein Längsschnitt durch das Rektum eines Menschen mit angrenzenden anatomischen Strukturen dargestellt. An das Rektum 16 schließt sich der Analkanal 17 an, wobei sich zwischen beiden die linea dentata 18 erstreckt. An dem Übergang vom Rektum zum Analkanal befindet sich in der Wand die Proktodäaldrüse, in deren Bereich sich vermehrt Fistelgänge bilden. Ferner ist den anatomischen Gegebenheiten entsprechend ein innerer Schließmuskel 20, ein äußerer Schließmuskel 21 und der musculus levator ani 22 dargestellt.

In der linken Hälfte sind exemplarisch zwei Fistelgänge 23, 24 dargestellt, die sich jeweils vom Bereich der Proktodäaldrüse 19 ausgehend bis zur äußeren Haut einer Pobacke 25 erstrecken. Während der Fistelgang 23 subkutan verläuft, erstreckt sich der Fistelgang 24 durch den inneren Schließmuskel 20.

In der rechten Hälte ist ein Fistelgang 26 dargestellt, welcher sich durch den inneren und den äußeren Schließmuskel 20, 21 erstreckt, das heißt in einem sogenannten transsphinkten Verlauf. Alle Fistelgänge haben eine vom Rektum 16 ausgehende innere Öffnung 27 und eine in der äußeren Pobacke 25 befindliche äußere Öffnung 28.

In den Fistelgang 26 ist ein Fistelblocker 1 eingezogen. Der Verschlusskörper 2 sitzt fest im Bereich der inneren Öffnung 27, wobei seine Anlagefläche 4 in dichtem Kontakt mit der Wand des Fistelganges 26 ist.

Der Applikationsstrang 11 erstreckt sich von dem Führungsabschnitt 6 des Verschlusskörpers 2 durch den Fistelgang 26 bis durch die äußere Öffnung 28 hindurch und ragt aus dieser nach außen hervor.

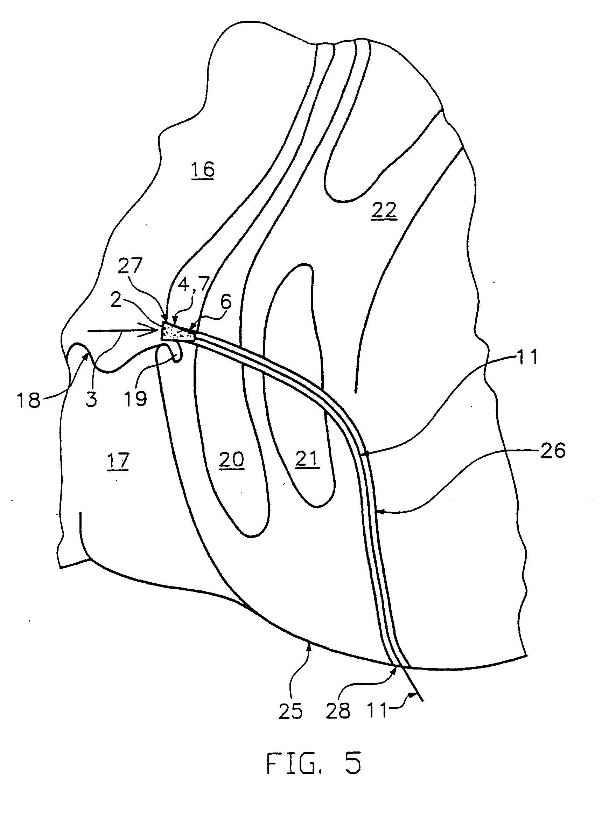

In Figur 5 ist eine vergrößerte Darstellung des rechten Teils von Figur 4 dargestellt. Gleiche Bezugszeichen bezeichnen gleiche Elemente, so dass diesbezüglich auf die vorstehenden Ausführungen verwiesen wird.

In Figur 5 steht das kaudale Ende des Verschlusskörpers 2 etwas in das Rektum 16 hervor. Dies Ende kann nach dem Einführen des Verschlusskörpers 2 z.B. mit einer geeigneten Zange abgetrennt werden, so dass der Verschlusskörper 2 bündig mit der inneren Wand des Rektums 16 endet. Der Verschlusskörper 2 entspricht der in Figur 1 dargestellten ersten Ausführungsform.

Der Applikationsstrang 11 erstreckt sich über die gesamte Länge des Fistelgangs bis zur äußeren Öffnung 28 und ragt etwa 1 bis 2 cm aus diesem hervor.

Im folgenden wird die Wirkungs- und Funktionsweise der in der Zeichnung dargestellten Ausführungsformen eines erfindungsgemäßen Fistelblockers näher erläutert.

Eine Fistel wird zunächst sondiert, das heißt es wird ein geeignetes stabähnliches Instrument von der äußeren Öffnung 28 in den Fistelgang vorgeschoben und der Verlauf der Fistel untersucht. Dies Instrument wird bis zum Austreten aus der inneren Öffnung 27 vorgeschoben. Anschließend wird von der inneren Öffnung 27 durch den Fistelgang 26 bis zur äußeren Öffnung 28 der Applikationsstrang hindurchgezogen bis der Verschlusskörper in der inneren Öffnung 27 steckt und der Führungsabschnitt 6 in den Fistelgang mündet. Der Verschlusskörper 2 wird soweit eingezogen bis er fest in dem Fistelgang 26 sitzt. Ein in das Rektum 16 hervorstehendes kaudales Ende kann wahlweise abgetrennt werden. Der Applikationsstrang 11 erstreckt sich wenige Zentimeter aus der äußeren Öffnung 28 hinaus.

Der Verschlusskörper 2 verschließt die innere Öffnung 27, so dass keine Kontamination des Fistelganges von innen erfolgt. Die Widerhakenelemente 14 arretieren den Verschlusskörper, so dass er nicht ungewollt in das Rektum 16 rutscht.

Der Verschlusskörper 2 bildet einen dichten Verschluss. Er kann semipermeabel ausgebildet sein, so dass Sekret aus dem Fistelgang durch ihn hindurch bis in das Rektum gelangen kann.

Es ist auch denkbar, dass der Schließabschnitt 7 an der Innenwand des Rektums über der inneren Öffnung 27, also außen angrenzend an den Fistelgang, dichtend anliegt.

Der Applikationsstrang dient als Drainageleitung. Er ist als Faden ausgebildet, entlang dem Sekrete, z.B. Eiter, in Folge der Dochtwirkung des Fadens durch die äußere Öffnung 28 nach außen geführt werden. Damit werden keimbildende Substanzen aus dem Fistelgang abgeführt, so dass dieser selbständig zuheilen kann. Der Applikationsstrang kann resorbierbar sein.

Besteht der Verschlusskörper 2 aus resorbierbarem Material, so kann er von dem Körper über einen längeren Zeitraum, ca. sechs bis zwölf Wochen, resorbiert werden. Dies wird besonders begünstig, wenn er eine poröse, schwammartige Oberflächenstruktur hat, so dass er von körpereigenen Substanzen auch von innen langsam umgebaut werden kann.

Besteht der Verschlusskörper 2 aus nicht resorbierbarem Material, so kann er über längere Zeit in dem Fistelgang verbleiben.

Je nach den anatomischen Gegebenheiten des Fistelgangs kann der Verschlusskörper 2 auch tiefer in dem Fistelgang eingezogen werden als in Figur 5 dargestellt.

Die zu der Fistelöffnung passende Größe des Verschlusskörpers 2 kann beispielsweise durch separate Probesitzschablonen ermittelt werden. Diese Probesitzschablonen entsprechen der Gestalt eines Verschlusskörpers 2, wie zu den drei Ausführungsformen beschrieben, gegebenenfalls ohne Widerhakenelemente 14. Es stehen mehrere Schablonen verschiedener Größe und Form zur Verfügung, so dass die genaue Passgröße für die jeweilige Fistel ermittelt wird und dementsprechend ein Fistelblocker 1 mit einem Verschlusskörper 2 passender Größe ausgewählt werden kann.

Der erfindungsgemäße Fistelblocker ermöglicht eine äußerst schonende Behandlung einer Fistel, bei der der invasive Eingriff herkömmlicher Behandlungen wesentlich reduziert wird, so dass Gewebe kaum verletzt wird und der Patient eine äußerst schonende Behandlung der Fistel erfährt.

## Patentansprüche

1. Fistelblocker (1) zum Sanieren eines Fistelganges (26), mit einem stöpselartigen, wenigstens ansatzweise in einen Fistelgang (26) einführbaren Verschlusskörper (2), weicher eine sich mindestens teilweise umfangsseitig, quer zur Einführrichtung (3) erstreckende und in Kontakt mit der Wand eines Fistelganges (26) bringbare Anlagefläche (4) aufweist, wobei der Verschlusskörper (2) mit einem flexiblen, in den Fistelgang (26) einführbaren Applikationsstrang (11) versehen ist, welcher als Drainageleitung ausgebildet ist.

2. Fistelblocker nach Anspruch 1,
**dadurch gekennzeichnet,**
**dass** der Verschlusskörper (2) einen in Einführrichtung (3) kranial angeordneten Führungsabschnitt (6) aufweist.

3. Fistelblocker nach Anspruch 1 oder 2,
**dadurch gekennzeichnet,**
**dass** der Verschlusskörper (2) einen in Einführrichtung (3) kaudal angeordneten, die Anlagefläche (4) aufweisenden Schließabschnitt (7) aufweist.

4. Fistelblocker nach einem der vorhergehenden Ansprüche,
**dadurch gekennzeichnet,**
**dass** der Verschlusskörper (2) etwa kegelförmig ausgebildet ist.

5. Fistelblocker nach einem der vorhergehenden Ansprüche,
**dadurch gekennzeichnet,**
**dass** der Verschlusskörper (2) eine konkave Außenform aufweist.

6. Fistelblocker nach einem der vorhergehenden Ansprüche,
**dadurch gekennzeichnet,**
**dass** der Verschlusskörper (2) etwa ei-förmig ausgebildet ist.

7. Fistelblocker nach einem der vorhergehenden Ansprüche,
**dadurch gekennzeichnet,**
**dass** die Länge des Verschlusskörpers (2) in Einführrichtung (3) etwa bis 2 cm, vorzugsweise 0,5 bis 1 cm, entspricht.

8. Fistelblocker nach einem der vorhergehenden Ansprüche,
**dadurch gekennzeichnet,**
**dass** der Verschlusskörper (2) aus resorbierbarem Material besteht.

9. Fistelblocker nach einem der vorhergehenden Ansprüche,
**dadurch gekennzeichnet,**
**dass** der Verschlusskörper (2) aus Polydioxanon, Polyglykolsäure und/oder Trimethylcarbonat besteht.

10. Fistelblocker nach einem der Ansprüche 1 bis 7,
**dadurch gekennzeichnet,**
**dass** der Verschlusskörper (2) aus Metall, vorzugsweise Titan, besteht.

11. Fistelblocker nach einem der vorhergehenden Ansprüche,
**dadurch gekennzeichnet,**
**dass** der Verschlusskörper im Inneren hohl ausgebildet ist.

12. Fistelblocker nach einem der vorhergehenden Ansprüche,
**dadurch gekennzeichnet,**
**dass** der Verschlusskörper (2) eine semipermeable Oberflächenstruktur, vorzugsweise als Membran, aufweist.

13. Fistelblocker nach einem der vorhergehenden Ansprüche,
**dadurch gekennzeichnet,**
**dass** der Verschlusskörper (2) eine schwammartige Struktur aufweist.

14. Fistelblocker nach einem der vorhergehenden Ansprüche,
**dadurch gekennzeichnet,**
**dass** der Verschlusskörper (2) im Inneren mit Kanälen (12) durchsetzt ist.

15. Fistelblocker nach einem der vorhergehenden Ansprüche,
**dadurch gekennzeichnet,**
**dass** der Verschlusskörper (2) mehrere über seine Oberfläche verteilte Vertiefungen (8) aufweist.

16. Fistelblocker nach einem der vorhergehenden Ansprüche,
**dadurch gekennzeichnet,**
**dass** der Fistelblocker (1) mit einer den Verschlusskörper (2) in einem Fistelgang (26) arretierenden Verankerungseinrichtung (13) versehen ist.

17. Fistelblocker nach Anspruch 16,
**dadurch gekennzeichnet,**
**dass** die Verankerungseinrichtung (13) mehrere die Bewegung entgegen der Einführrichtung (3) blockierende Widerhakenelemente (14) aufweist.

18. Fistelblocker nach Anspruch 17,
**dadurch gekennzeichnet,**
**dass** die Widerhakenelemente (14) begrenzt bewegbar, sich selbst seitlich abspreizend gelagert sind.

## Claims

1. Fistula blocker (1) for clearing up a fistula passage (26) with a plug-like closure device (2) which can at least to some extent be inserted into the fistula passage (26) and which has a bearing surface (4) extending at least partially circumferentially and perpendicular to the direction of insertion (3) and which can be brought into contact with the wall of the fistula passage (26), and where the closure device (2) is provided with a flexible application string (11) which can be inserted into the fistula passage (26), the application string being designed as a drainage pipe.

2. Fistula blocker according to Claim 1,
**characterized in that**
the closure device (2) has a guide section (6) laid out cranially in the direction of insertion (3).

3. Fistula blocker according to Claims 1 and 2,
**characterized in that**
the closure device (2) has a closure section (7) having a bearing surface (4) laid out caudally in the direction of insertion (3).

4. Fistula blocker according to one of the previous Claims,
**characterized in that**
the closure device (2) is formed conically.

5. Fistula blocker according to one of the previous Claims,
**characterized in that**
the closure device (2) has a concave outer shape.

6. Fistula blocker according to one of the previous Claims,
**characterized in that**
the closure device (2) is somewhat shaped like an egg.

7. Fistula blocker according to one of the previous Claims,
**characterized in that**
the length of the closure device (2) corresponds in the direction of insertion (3) to about 2 cm, preferably from 0.5 cm to 1 cm.

8. Fistula blocker according to one of the previous Claims,
**characterized in that**
the closure device (2) is made of reabsorptive material.

9. Fistula blocker according to one of the previous Claims,
**characterized in that**
the closure device (2) is made out of poly-dioxanone, poly-glycolic acid and/or trimethyl-carbonate.

10. Fistula blocker according to one of the Claims 1 through 7,
**characterized in that**
the closure device (2) is made out of metal, preferably titanium.

11. Fistula blocker according to one of the previous Claims,
**characterized in that**
the closure device is hollowed out on the inside.

12. Fistula blocker according to one of the previous Claims,
**characterized in that**
the closure device (2) has a semi-permeable surface structure, preferably of membrane.

13. Fistula blocker according to one of the previous Claims,
**characterized in that**
the closure device (2) has a spongy structure. ,

14. Fistula blocker according to one of the previous Claims,
**characterized in that**
the closure device (2) is riddled on the inside with channels (12).

15. Fistula blocker according to one of the previous Claims,
**characterized in that**
the closure device (2) has several indentations (8) spread out over its surface.

16. Fistula blocker according to one of the previous claims,
**characterized in that**
the fistula blocker (1) is provided with an anchoring device (13) for locking the closure device (2) tight in a fistula passage (26).

17. Fistula blocker according to Claim 16,
**characterized in that**
the anchoring device (13) has several barbed sections (14) blocking its movement contrary to the direction of insertion (3).

18. Fistula blocker according to Claim 17,
**characterized in that**
the barbed sections (14) are restricted in their flexibility, and laid out shored up laterally.

## Revendications

1. Obturateur de fistule pour assainir un canal de fistule (26), comportant un corps d'obturation (2) de type bouchon, pouvant être introduit au moins comme garniture dans un canal de fistule (26) et qui présente une surface de contact (4) s'étendant au moins en partie périphériquement, transversalement à la direction d'introduction (3) et pouvant être amenée en contact avec la paroi d'un canal de fistule (26), le corps d'obturation (2) étant pourvu d'un cordon d'application (11) flexible, pouvant être introduit dans le canal de fistule (26) et qui est réalisé à la manière d'un conduit de drainage.

2. Obturateur de fistule selon la revendication 1, **caractérisé en ce que** le corps d'obturation (2) comporte une section de guidage (6) disposée côté tête dans la direction d'introduction (3).

3. Obturateur de fistule selon la revendication 1 ou 2, **caractérisé en ce que** le corps d'obturation (2) comporte une section de fermeture (7) disposée côté queue dans la direction d'introduction (3) et qui présente la surface de contact (4).

4. Obturateur de fistule selon l'une des revendications précédentes, **caractérisé en ce que** le corps d'obturation (2) est réalisé de forme sensiblement conique.

5. Obturateur de fistule selon l'une des revendications précédentes, **caractérisé en ce que** le corps d'obturation (2) présente une forme extérieure concave.

6. Obturateur de fistule selon l'une des revendications précédentes, **caractérisé en ce que** le corps d'obturation (2) est réalisé sensiblement en forme d'oeuf.

7. Obturateur de fistule selon l'une des revendications précédentes, **caractérisé en ce que** la longueur du corps d'obturation (2) dans la direction d'introduction (3) correspond approximativement à 2 cm, de préférence de 0,5 à 1 cm.

8. Obturateur de fistule selon l'une des revendications précédentes, **caractérisé en ce que** le corps d'obturation (2) est constitué d'une matière se résorbant.

9. Obturateur de fistule selon l'une des revendications précédentes, **caractérisé en ce que** le corps d'obturation (2) est en polydioxanone, acide polyglycolique et/ou triméthylcarbonate.

10. Obturateur de fistule selon l'une des revendications 1 à 7, **caractérisé en ce que** le corps d'obturation (2) est en métal, de préférence en titane.

11. Obturateur de fistule selon l'une des revendications précédentes, **caractérisé en ce que** le corps d'obturation est réalisé creux à l'intérieur.

12. Obturateur de fistule selon l'une des revendications précédentes, **caractérisé en ce que** le corps d'obturation (2) présente une structure superficielle semi-perméable, de préférence sous la forme d'une membrane.

13. Obturateur de fistule selon l'une des revendications précédentes, **caractérisé en ce que** le corps d'obturation (2) présente une structure de type éponge.

14. Obturateur de fistule selon l'une des revendications précédentes, **caractérisé en ce que** le corps d'obturation (2) est traversé à l'intérieur par des canaux (12).

15. Obturateur de fistule selon l'une des revendications précédentes, **caractérisé en ce que** le corps d'obturation (2) présente plusieurs renfoncements (8) répartis sur sa surface.

16. Obturateur de fistule selon l'une des revendications précédentes, **caractérisé en ce que** l'obturateur de fistule (1) est pourvu d'un dispositif d'ancrage (13) bloquant le corps d'obturation (2) dans un canal de fistule (6).

17. Obturateur de fistule selon la revendication 16, **caractérisé en ce que** le dispositif d'ancrage ( 13) comporte plusieurs éléments à barbules (14) bloquant le mouvement dans le sens contraire au sens d'introduction (3).

18. Obturateur de fistule selon la revendication 17, **caractérisé en ce que** les éléments à barbules (14) sont montés déplaçables dans une mesure limitée et de façon à s'écarter eux-mêmes latéralement.
